## Europäisches Patentamt

## European Patent Office

## Office européen des brevets

(11) Publication number:

# 0 119 768
A1

## (12) EUROPEAN PATENT APPLICATION

(21) Application number: 84301176.8

(22) Date of filing: 23.02.84

(51) Int. Cl.³: **C 12 N 11/08,** C 12 P 1/00, C 05 F 11/10

---

(30) Priority: 23.02.83 GB 8304997

(43) Date of publication of application: 26.09.84 Bulletin 84/39

(84) Designated Contracting States: **DE FR GB IT NL**

(71) Applicant: **Kent Scientific and Industrial Projects Limited, The Physics Laboratory The University, Canterbury Kent CT2 7NR (GB)**

(72) Inventor: **Burns, Richard George, 19, Lichfield Avenue, Canterbury Kent (GB)**
Inventor: **Sarker, Jawed Mansab, 245, Water Gardens, Edgware London W.2. (GB)**

(74) Representative: **Baillie, Iain Cameron et al, c/o Ladas & Parry Isartorplatz 5, D-8000 München 2 (DE)**

(54) Immobilisation of enzymes.

(57) Enzymes for example cellulases are immobilised by complexing with polymers derived from quinones which polymers resemble humic polymers. Preferably the quinone is formed in the presence of the enzyme and self-polymerises to incorporate the enzyme. The resulting immobilised enzyme polymer complex can be further stabilised by supports such as activated clays. The resulting immobilised enzyme complex is particularly suitable for incorporation in soil but can be used for other enzymatic processes.

EP 0 119 768 A1

-1-

IMMOBILISATION OF ENZYMES

This invention relates to the immobilisation of enzymes particularly of enzymes on polymeric supports.

Under natural conditions the most stable enzymes are usually bound, for example to membranes within a cell or on cell walls. With the extraction of enzymes there has been considerable study of immobilisation of enzymes isolated from living organisms on or within solid supports, for example polystyrene, porous glass, or silica-alumina. For commercial applications of enzymes these immobilised-enzyme systems often are advantageous as compared to free enzymes in solution. For example the enzyme activity will have greater longevity while an enzyme bound to a solid phase is more easily recoverable from a reaction mixture than a soluble counter-part. Enzymes can be bound to substrates by chemical methods in which covalent bonds are formed with the enzyme or by physical methods where weaker interactions or physical entrapment of the enzyme is involved. Thus enzymes have been attached to such synthetic supports as acrylamide-based polymers and polypeptides and natural supports such as cellulose or starch. Where a monomer for a polymeric substrate is multi-functional, then presence of the enzyme at the beginning of a polymerisation reaction can result in a covalently bound enzyme incorporated into the structure of the polymer and distributed through the polymer mass.

In the soil many extra-cellular enzymes are immobilised in soil by association with macro-molecular structures sometimes known as humic polymers. In common with other systems this provides a stability which is not apparent with the free enzymes which are rapidly de-natured, degraded or otherwise inactivated in soil. These humic macro-molecular structures are extremely complex in nature and appear to be derived from plant residues such as lignins or from microbial synthesis. Because of the importance of these humic structures in soil fertility there have been various studies of the structures of humates and their possible formation in the soil. However, there has been relatively little commercial interest in synthesising such polymers.

In Rowell et al, Soil Biol. Biochem Vol. 5 pp 699-703 Pergamon Press 1973, there is mention of complexes of p-benzoquinone and enzymes but this does not indicate formation of a polymer with which the enzyme is complexed.

It has now been found that certain quinone polymers which resemble humic polymeric structures, can provide a polymeric complex structure in which the enzyme is immobilised particularly if the polymer is formed in the presence of the enzymes to be immobilised. Because of the resemblance these final structures to certain natural humic polymer structures the products are useful in a variety of functions particularly but not limited to the treatment of soils and plant residues.

According to the invention therefore there is provided an immobilised enzyme synthetic polymer structure comprising polymerised units of at least one quinone complexed with at least one enzyme.

The invention also provides a method for preparing immobilised enzyme polymer structures wherein at least one quinone polymerises in the presence of an enzyme to form a synthetic polymer enzyme complex.

It can be speculated that, in a preferred aspect of the invention, the chemical interaction between the polymerising quinone and the enzyme structure produces particularly strong covalent bonds so that, in the resulting product, the immobilisation of the enzyme is especially effective as compared to other structures in which the enzyme is more loosely held. Thus the preferred product can be superior to one which is prepared by forming the polymer and then placing the formed polymer in contact with an enzyme. Although, in the latter case there will be adsorption of enzyme onto such a pre-formed polymer and possibly the formation of bonding, the stability may not be as strong as the preferred aspect. As compared to other organic polymer structures, the relatively mild conditions under which the polymer of the present invention is formed minimises any possible interference with, or degradation of, the enzyme structure or other inactivation. Also, since the resulting polymer structure in many ways resembles natural humic polymers, the product is particularly useful in applications extremely stable immobilised enzymes are important, for instance in the treatment of soil. However, the applications of the polymer are not limited to such uses.

- 3 -

The monomeric materials from which the polymers are formed comprise a wide variety of quinone materials and aromatic materials having quinone structures. Thus the basic ring structure to which the quinone groups are attached and which can also include polycyclic structures can be substituted by many other groupings as is well known in the art. For example certain amino acids such as tyrosine will demonstrate quinonic structures on appropriate treatment. Certain polyhydroxy phenols and quinones which have been used in the study of humic type polymers will, however, be possibly unsuitable because of chemical interference with the activity of some enzymes. In general such interference can be predicted from a knowledge of the chemical properties of the quinone or phenol in question but the undesirability of a particular starting quinone or phenol and the enzyme in question can readily be determined by simple testing of the activity of the final product. Thus p-benzoquinone has not proved suitable.

In a preferred form of the invention the quinone is produced from a phenol and during the course of the production of the quinone and its subsequent polymerisation the enzyme is introduced so that it is present in the system during the simultaneous production of quinone and subsequent polymerisation.

Polyhydroxy phenols and similar compounds such as hydroxybenzoic acids which can be employed include L-tyrosine, pyrogallol, resorcinol, orcinol, phloroglucinol, 2,4-di-hydroxybenzoic acid, 3,5-dihydroxybenzoic acid, catechol, protocatechuic acid, ferulic acid, caffeic acid, and p-hydroxybenzoic acid. Co-polymers involving two or more of these phenolic compounds can be employed for example hetero-polymers of resorcinol and pyrogallol.

The polymerisation is readily initiated because of the tendency to self-polymerisation of these quinones and indeed usually polymerisation will commence as the quinones are formed from the phenols. Generally slightly acidic conditions are preferred for the initiation of the polymerisation and a range of pH 5 to 7 is preferred. In the preferred method the quinone will be produced by oxidation of the corresponding phenol in a buffer providing slightly acid system in

- 4 -

which the polymerisation then continues. The production of quinones from phenols can be by various conventional methods. For example a phenol and hydrogen donor can be combined and passed into an oxidising system for example an enzymatic oxidising system. A suitable hydrogen donor is hydrogen peroxide. After the reaction has started addition of the enzyme to be combined into the system can then be commenced. This reaction involving subjecting the phenol to an oxidising system particularly an enzymatic oxidising system with simultaneous addition of the enzyme desird for incorporation into the complex has been found to give products which are particularly stable. Thus enzyme oxidising systems using peroxidase or tyrosinase have been particularly effective.

The reaction is generally in a buffered system the product being obtained as a colloidal dispersion in the aqueous system in which the reaction is carried out. The final polymer complex can be then separated from the system by concentration, dialysis and freeze drying. Certain polymeric complexes will be particulate and therefore somewhat easier to separate. The molecular weight is approximately from 10,000 to 100,000.

The enzymes which can be immobilised within the products of the present invention and by the method of the invention comprise a wide variety of enzymes presently used for investigation and for commerical purposes. Hydrolases particularly cellulases (such as $\beta$-D-glucosidase or cellulase preparations from <u>Trichderma</u> <u>viride</u> or <u>Aspergillus</u> <u>niger</u>), endo-and exo-cellulases and urease can be employed. Other groups of enzymes such as oxidoreductases, transferases, ligases, lyases, and isomerases may also be employable. In case of some enzymes it will be readily apparent if they can be employed because naturally occurring, humic structures are known which contain these enzymes. However, for any particular enzyme there may be the possibility of steric interference which would hinder the effectiveness of the enzyme. The suitability of any given enzyme can, to some extent, be predicted from experience with other methods of immobilisation or at most a simple test would reveal whether use in the present invention might yield an active product.

- 5 -

Generally the method of preparation will be such that the enzyme is incorporated into the polymer as it is formed. In some instances the nature of the polymeric structure may be such that the basic polymer can be formed and a particular enzyme complexed thereto after formation of the polymer. A polymer formed from certain amino acids such as L-tyrosine might be particularly suitable for such a technique.

The polymeric complex formed according to the invention can be further stabilised by linking to other supports. Other types of supports could be organic polymeric structures, silica and other materials already known for the immobilisation of enzymes. Thus the polymers could be incorporated into inorganic structures such as alumina or clays, ion exchange resins, natural organic materials such as starch or into gel systems and into micro-encapsulation systems. Thus the polymer complex can be tightly adsorbed onto a DEAE cellulose or onto a bentonite clay. Particularly preferred would be adsorption of the polymer complex onto a clay activated, for example, by cyanuric chloride. Polymer complexes immobilised on ultrafiltration membranes would be particularly useful in small scale reactors.

The resulting complex polymers whether by themselves or further stabilised on a different support can be employed in soil. The enzyme complex can thus enhance, replace or supplement natural enzymatic action arising from enzymes that are present in soil. The incorporation into the soil of the polymer enzyme complexes of the invention may be used to improve soil structure and to accelerate plant nutrient solubilisation or to remove by bio-degradation possible pollutants or agricultural chemicals which have served their function. The polymer enzyme complexes can also be used in other enzymatic systems for example fermenters and columns in which they may be particularly effective because of the resemblance of the polymers of the present invention to certain natural polymers. This cellulase can be employed for enzymatic treatment of natural cellulosic materials for example paper or straw.

The invention will now be particularly described in the following examples.

0119768

- 6 -

Example 1.

250mg resorcinol was dissolved in 100 ml acetate buffer of pH 5.5 and the resulting solution placed in one cylinder of a gradient mixer. To the other cylinder of the mixer 1% by volume hydrogen peroxide was added. The resorcinol solution and hydrogen donor were mixed and passed slowly by drops into a solution of peroxidase (1mg ml$^{-1}$ in acetate buffer) in a vessel surrounded by ice. After 2 hours a solution of $\beta$-D-glucosidase (40mg in an acetate buffer of pH 5.5) was gradually added over 12 hours. The $\beta$-D-glucosidase is $\beta$-D-glucosidase-sweet almond (EC 3.2.1.21). The final product was an aqueous dispersion of a brown polymer. This dispersion was concentrated under a vacuum at 23$^{\circ}$C, dialysed and freeze dried. 150mg of enzyme polymer complex was obtained which was colloidal at pH 6.0 or above and flocculated at pH 2.0

A portion of the complex was combined with fresh soil and incubated at 25$^{\circ}$C for 21 days. An amount of free $\beta$-D-glucosidase corresponding to that in the polymer was incorporated in a separate sample of soil. The free $\beta$-D-glucosidase was rapidly inactivated and after 21 days there was a 80% loss of the original activity in the free $\beta$-D-glucosidase. The polymer enzyme complex of the present invention did not demonstrate any loss in enzyme activity until the 9th day and activity thereafter declined only slowly with 80% of the original activity remaining on the 21st day.

Example 2

100 g of bentonite was suspended in 200ml dioxane and 5g cyanuric chloride was added. This was agitated at 20$^{\circ}$C for 2 hours. The bentonite-cyanuric chloride complex was separated by centrifuging and washed several times with dioxane and then dried under vacuum. The activated clay and polymeric complex and Example 1 were then combined in a proportion of 30mg polymer enzyme complex to 1g clay. The resulting solid clay/polymer enzyme complex was then employed in a column through which was passed cellulosic material for break-down by the enzyme. After ten cycles including washing between cycles, the clay/polymer enzyme complex retained at least 65% of the initial activity.

0119768

- 7 -

Samples of the polymer/enzyme complexes of Examples 1 and 2 were exposed to a universal buffer system, components of which do not inhibit enzyme activity. This exposure was at various pH values but at constant ionic strength. A sample of free $\beta$-D-glucosidase had an optimum activity at pH 5.6 the enzyme/polymer complex had an optimum activity at pH 5.9. A sample of resorcinol polymer fixed on activated bentonite clay in accordance with Example 2 demonstrated at least 20-fold increase in activity as compared to pyrogallol polymer enzyme complex fixed on simple bentonite. $\beta$-D-glucosidase directly fixed on bentonite gave a structure without any activity.

Example 3.

Pyrogallol and resorcinol were separately dissolved in an acetate buffer of pH 5.5 and mixed to provide a total of 250mg of phenols. A polymer enzyme complex of $\beta$-D-glucosidase was then produced in accordance with the procedure of Example 1 excepting that the mixture of pyrogallol and resorcinol was used instead of the solution of resorcinol. The resulting polymer was brown-black and colloidal at pH 6 and flocculated at pH 2.0.

Example 4

Tyrosine/$\beta$-D-glucosidase complex.

2g L-tyrosine was dissolved in 50ml phosphate buffer of pH 7.0 and the mixture was incubated with a phenol oxidase (tyrosinase). $\beta$-D-glucosidase was added to the reaction mixture over a period of 12 hours during which the quinones derived from tyrosine polymerised to give rise to an enzyme synthetic polymer structure. The product was dark-coloured and was washed several times with water, dialysed and lyophilised before use.

Polymer complex of Examples 1, 3 and 4 was exposed to protease (streptomyces griseus) and separately a sample of free enzyme was exposed to protease. A sample of bentonite/polymer enzyme complex prepared as in Example 2 was also exposed to protease. After 24 hours there was a 70% reduction in the activity of the free enzyme and only 15% loss in the activity of the polymer/enzyme complex. The bentonite polymer/enzyme complex did not show any loss of enzyme activity.

Samples of the above enzyme tyrosine complex of Example 4 and enzyme/resorcinol complex of Example 1 and resorcinol/pyrogallol complex of Example 3 as well as a sample of free $\beta$-D-glucosidase were subjected to temperatures from 4°C to 75°C for one hour. The free $\beta$--D-glucosidase rapidly lost activity particularly above 40°C . The polymer complexes of the invention demonstrated resistance to temperatures up to 70°C although greater resistance was demonstrated by the L-tyrosine complex.

L-tyrosine complex was exposed to protease in accordance with Example 2. simultaneously with exposure of free $\beta$-D-glucosidase. The L-tyrosine polymer complex showed only a 20% loss in enzyme activity over 24 hours exposure as compared to the loss of activity of 70% for free glucosidase.

Samples of free $\beta$-D-glucosidase and complex with L-tyrosine were subjected to methanol, butanol and acetone by applying mixtures of the organic solvent and water. Between 0 and 20% of solvent caused a drop of 75% approximately of original activity for the free glucosidase compared to a drop to 50% or less for the complexes. The increase in the proportion of solvent did not significantly affect the activity for the complex but with the free enzyme the loss of activity increased.

Example 5

250mg resorcinol was dissolved in 100 ml of 100 mM acetate buffer of pH 5.4 and the resulting solution placed in one cylinder of a gradient mixer. To the other cylinder of the mixer 1% weight by volume hydrogen peroxide was added. The resorcinol solution and hydrogen donor were mixed 1:1 and passed slowly by drops into a solution of peroxidase (10mg-ml of 100 mM acetate buffer pH5.4) in a vessel surrounded by ice. After 2 hours a solution of $\beta$-D-glucosidase (40mg in 10ml of the same acetate buffer) was gradually added over 10 hours. The $\beta$-D-glucosidase is $\beta$-D-glucosidase-sweet almond (Sigman Chemical Company, England). The final product was an aquous dispersion of a brown polymer. This dispersion was concentrated under a vacuum at 23°C, dialysed against distilled water and freeze dried.

The free enzyme and the above enzyme polymer complex were separately immobilised on :

(i)     K-carrageenan (Sansho Co. Ltd., Japan) using the technique of Takata et al (1982) J. Ferment, Technol. 60. 431-437.

(ii)    DEAE cellulose (Whatman Limited, England  D.E. 52) using the technique of Sarkar and Mayaudon (1983) Biotechnol. Letts. 5, 201-206

(iii)   Ultrafiltration Membrane (PM 10 Amicon Corp. U.S.A.) in an ultrafiltration cell (also supplied by Amicon Corp.) by adding 5mg of emzyme or polymer/enzyme complex in 10ml of 100mM acetate buffer (pH 5.4) to the cell and filtering under low pressure and washing with buffer.

Measurement of enzyme activity using p-nitrophenyl-$\beta$-D-glucopyranoside showed that immobilisation on ultrafiltration membranes gave a combination of high $V_{max}$ and low $K_m$ values in comparison with immobilisation on K-carrageenan and DEAE cellulose.

The thermostability of the enzyme/polymer complex was superior to that of the free enzyme in relation to all three supports and the immobilised forms of enzyyme polymer complex were superior to the un-immobilised form of complex.

When a cell containing enzyme/polymer complex immobilised on a membrane was subjected to seven cycles of adding substrate, removing product and washing the decrease was only 20% while variation in flowrate through the cell of p-nitrophenyl-$\beta$-D-glucopyranoside of from 0.1 ml/min to 0.75ml/min did not decrease product formation.

Example 6

Polymer complexes were prepared in accordance with Example 1 but employing instead of resorcinol, the following :-

phloroglucinol, orcinol, catechol, protocatechuic acid, caffeic cid, ferulic acid, 2,4-dihydroxybenzoic acid, 3,5-dihydroxybenzoic acid and p-hydroxy-benzoic acid.

All the copolymers were brown/black in colour, had a pH of 5.2 and flocculated upon addition of 1M H Cl. The complexes were less active than the free enzyme. Thus while $\beta$-D-glucosidase-resorcinol copolymer (e.g. of Example 1) retained 55% of the activity of the free enzyme; 2,4-dihydroxybenzoic acid retained 32%; 3,5-dihydroxybenzoic acid 29%; phloroglucinol, 27%; ferulic acid and L-tyrosine, 25%, catechol, 21%; caffeic acid and pyrogallol, 19%; orcinol, 15% protocatechuic acid, 14%.

Example 7

Following the procedure of Example 1 polymer complexes were prepared from various polyhydroxy phenols using a cellulose preparation from Aspergillus Niger (EC 3.2.1.4). The properties of the copolymer complexes are listed below. Each of the complexes had a lower activity than the free enzyme but were more resistant to deactivation than the free enzyme particularly in regard to heat stability and effect of protease.

Enzymatic Activity

| PHENOLIC POLMYER COMPONENT | MOL.WT | $K_m(mM)$ | $V_{max}$* |
|---|---|---|---|
| Resorcinol | 110.1 | 6.2 | 2.4 |
| Pyrogallol | 126.1 | 4.6 | 1.8 |
| Phloroglucinol | 162.1 | 4.9 | 3.0 |
| Orcinol | 124.1 | 7.8 | 2.1 |
| Protocatechuic Acid | 154.1 | 4.2 | 1.2 |
| Ferulic Acid | 194.2 | 2.1 | 1.1 |
| 2,-Dihydroxybenzoic Acid | 154.1 | 6.8 | 1.7 |
| 3,5-Dihydroxybenzoic Acid | 154.1 | 8.1 | 2.1 |
| p-Hydroxybenzoic Acid | 138.1 | 2.1 | 1.6 |
| Cellulase Alone | - | 8.5 | 4.5 |

*umol of glucose released $mg^{-1}h^{-1}$ of copolymer.

- 11 -

ANALYSIS DATA

All the synthetic copolymers were brown/black in colour, had a pH of 6.2, and like humic acids, flocculated upon addition of 1M-HCl. Both UV and visible spectra showed no maximum or minimum absorption and optical density decreased as wavelength increased. Humic acids and humic-enzyme extracts have the same characteristics. The ratio of optical densities at 465nm and 665nm (i.e. $E_4/E_6$ ratio - Schnitzer M., 1978 Humic Substatnces: Chemistry and Reactions in Soil Organic Matter (M. Schnitzer and S.U. Khan Eds.)., pp 13-14 Elsevier, New York) for the $\beta$-D-glucosidse-phenolic copolymers were: resorcinol 5.2; L-tyrosine 5.8; pyrogallol 8.7; and resorcinol/pyrogallol 7.9. For comparison, humic enzyme complex extracted from soil and known to conain a mixture of humic and fulvic acids had an $E_4/E_6$ ratio of 8.3 Humic acids extracted from soils have $E_4/E_6$ ratios from approximately 3.5 to 5.8 according to soil type whilst fulvic acid ratios tend to be higher (i.e. 7.6-11.2). The $E_4/E_6$ ratios for reorcinol and L-tyrosine copolymers are comparable to those of humic acids and indicate a relatively high degree of condensation. In contrast the $E_4/E_6$ ratios of pyrogallol, resorcinol/pyrogallol and the humic-enzyme extract are close to those of fulvic acid. C,H,N,O and S analysis of the $\beta$-D-glucosidase-phenolic copolymers is compared with a humic-enzyme complex extracted from soil and some typical analyses of humic acid an fulvic acid taken from the literature. The low carbon and high oxygen contents of all the copolymers was more characteristic of fulvic cids than humic acids. L-tyrosine-containing copolymers were significantly different from the other three copolymers in C,O and N content but similar to the humic enzyme extrct.

The IR spectra of all the $\beta$-D-glucosidase-phenolic copolymers and the humic-enzyme complex extracted from soil showed distinct absorption bands at the following frequencies: $3400cm^{-1}$ (hydrogen-bonded OH groups), 2920 and $2960^{-1}$ (aliphatic C-H), $1600cm^{-1}$ ($-COO^-$, aromatic C=C ) and $1400cm^{-1}$ ($-COO^-$ aliphatic C-H). These spectra provided information concerning the distribution of functional groups in $\beta$-D-glucosidase-phenolic copolymers and, confirms that these synthetic polymers are analogous to native soil humic substances.

0119768

— 12 —

CLAIMS:

1.     An immobilised enzyme comprising synthetic polymer of at least one quinone moiety and complexed therewith at least one enzyme.

2.     An immobilised enzyme according to claim 1 in which the quinone is derived from a polyhydroxy phenol.

3.     An immobilised enzyme according to claim 1 in which the quinone is derived from a phenolic amino acid.

4.     An immobilised enzyme according to any one of claims 1 to 3 which is fixed to a different enzyme-immobilising support.

5.     An immobilised enzyme according to claim 4 in which the support is a clay.

6.     A method for immobilising enzymes comprising forming a complex between a synthetic polymer of a quinone and an enzyme.

7.     A method according to claim 6 wherein the enzyme is dispersed in a aqueous system with the quinone which is subjected to polymerising conditions.

8.     A method according to claim 6 in which quinone is formed from a corresponding phenol and enzyme is present as the quinone is formed.

9.     A method according to claim 8 in which the phenol is subjected to oxidising conditions in a slightly acidic environment in the presence of the enzyme.

10.     A method according to claim 9 in which the oxidising conditions are those of an oxidising enzyme system.

11.     A method according to any one of claims 6 to 10 in which the enzyme/polymer complex when formed is combined with a further different enzyme immobilising support.

12. A method according to claim 11 in which the further support is a clay.

13. A method for effecting an enzymatic reaction wherein a reactant is exposed to an immobilised enzyme according to any one of claims 1 to 5 or an immobilised enzyme prepared according to a method according to any one of claims 6 to 12.

14. A method according to claim 13 in which the immobilised enzyme is added to a soil.

European Patent Office

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| D,X | CHEMICAL ABSTRACTS, vol. 79, no. 25, 24th December 1973, page 240, no. 145355k, Columbus, Ohio, US M.J. ROWELL et al.: "Enzymically active complexes of proteases and humic acid analogs" & SOIL BIOL. BIOCHEM. 1973, 5(5), 699-703 * Abstract * | 1,6,7, 13 | C 12 N 11/08 C 12 P 1/00 C 05 F 11/10 |
| Y | IDEM | 2,8-10 ,14 | |
| Y | CHEMICAL ABSTRACTS, vol. 66, no. 21, 22nd May 1967, page 8800, no. 94242t, Columbus, Ohio, US J.N. LADD et al.: "Comparison of some properties of soil humic acids and synthetic phenolic polymers incorporating amino derivatives" AUSTRALIAN J. SOIL RES. 4(1), 41-54, 1966 * Abstract * | 2,8,14 | **TECHNICAL FIELDS SEARCHED (Int. Cl. ³)** C 12 N C 05 F |
| Y | CHEMICAL ABSTRACTS, vol. 72, no. 15, 13th April 1970, page 354, no. 78600b, Columbus, Ohio, US I. LINDQVIST et al.: "Formation of humic acids by oxidation of phenolic compounds" & LANTBRUKS-HOEGSK. ANN. 1969, 35(4), 815-22 * Abstract * | 2,9,10 | |

-/-

The present search report has been drawn up for all claims

| Place of search THE HAGUE | Date of completion of the search 29-05-1984 | Examiner RYCKEBOSCH A.O.A. |
|---|---|---|

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82

0119768

European Patent Office

**EUROPEAN SEARCH REPORT**

Application number

EP 84 30 1176

| | DOCUMENTS CONSIDERED TO BE RELEVANT | | | Page 2 |
|---|---|---|---|---|
| Category | Citation of document with indication, where appropriate, of relevant passages | Relevant to claim | | CLASSIFICATION OF THE APPLICATION (Int. Cl. 3) |
| Y | CHEMICAL ABSTRACTS, vol. 89, no. 23, 4th December 1978, page 524, no. 196193v, Columbus, Ohio, US S.P. MATHUR et al.: "A chemical and spectroscopic characterization of some synthetic analogs of humic acids" & SOIL SCI. SOC. AM. J. 1978, 42(4), 591-6 * Abstract * | 2,14 | | |
| | ----- | | | |
| | | | | TECHNICAL FIELDS SEARCHED (Int. Cl. 3) |

The present search report has been drawn up for all claims

| Place of search THE HAGUE | Date of completion of the search 29-05-1984 | Examiner RYCKEBOSCH A.O.A. |
|---|---|---|

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO Form 1503. 03 82